# EUROPEAN PATENT APPLICATION

(11) **EP 4 015 581 A1**
(43) Date of publication of application: **22.06.2022**
(21) Application number: 20214116.4
(22) Date of filing: 15.12.2020
(51) Int. Cl.: C08L 83/12

(54) **EMULSIFIER SYSTEM**

(71) Applicant: Evonik Operations GmbH, 45128 Essen (DE)
(72) Inventor: AMAJJAHE, Sadik, 40591 Düsseldorf (DE); BERGFRIED, Stefan, 45359 Essen (DE); FRIEDRICH, Achim, 45529 Hattingen (DE)
(74) Representative: Evonik Patent Association

(57) **Abstract**

The invention relates to mixture compositions comprising A) crosslinked organopolysiloxanes having organopolysiloxane units linked by a building block containing polyether groups and B) crosslinked organopolysiloxanes having organopolysiloxane units bridged by a bridging siloxane and the use of these mixture compositions.

## Description

### Field of the Invention

The invention relates to mixture compositions comprising A) crosslinked organopolysiloxanes having organopolysiloxane units linked by a building block containing polyether groups and B) crosslinked organopolysiloxanes having organopolysiloxane units bridged by a bridging siloxane and the use of these mixture compositions.

### Prior Art

WO2019096593A1 discloses polymers and their use for dispersing solids pigments. EP2301987A1 discloses crosslinked organopolysiloxanes having organopolysiloxane units linked by a building block containing polyether groups.

WO2009138306 discloses crosslinked organopolysiloxanes having organopolysiloxane units bridged by a bridging siloxane.

There is still a need for superior emulsifier systems, especially for stabilizing solid particles in formulations.

### Description of the Invention

Surprisingly, it has been found that mixture compositions described in Claim 1 are high-performance emulsifiers, particularly for cosmetic, dermatological or pharmaceutical formulations, which are able to achieve this object.

The invention therefore provides to mixture compositions comprising A) crosslinked organopolysiloxanes having organopolysiloxane units linked by a building block containing polyether groups and B) crosslinked organopolysiloxanes having organopolysiloxane units bridged by a bridging siloxane as described in Claim 1.

A particular advantage of the mixture compositions according to the instant invention is its outstanding ability to stabilize pigment and particle containing systems e.g. in decorative cosmetics or in sun protection. Moreover, the invention enables the stabilization of a broad range of further W/O-formulations with different textures or containing versatile compounds challenging to formulate such as organic UV-filters. A special feature is the light and pleasant skin feeling in comparison to other emulsifiers of similar kind.

The mixture composition according to the instant invention comprises
A) crosslinked organopolysiloxanes having organopolysiloxane units linked by a building block of the formula (I)

   -CH₂-CH₂-(G)ₙO(EO)ₓ(PO)_{y}(XO)_{z}- formula (I),

   where
   G = divalent organic radical, preferably CH₂, C=O, CR¹₂ or CHR¹, preferably CH₂,
   EO = C₂H₄O
   PO = C₃H₆O
   XO = C₂H₃R¹O
   n = 1 to 16, preferably 1 to 9 and more preferably 1
   x = 2 to 50, preferably 5 to 30, preferably from 6 to 15,
   y = 0 to 50, preferably 0 or > 0 to 15, preferably 0,
   z = 0 to 10, preferably 0 or > 0 to 2, preferably 0,
   R¹ = independently of one another, identical or different radicals selected from the group comprising
   linear or branched, saturated or unsaturated alkyl radicals having 1 to 30 carbon atoms, which are optionally interrupted by ether functions, preferably alkyl radicals having 1 to 14 carbon atoms, alkaryl radicals having 7 to 18 carbon atoms, and
   aryl radicals having 6 to 16 carbon atoms,
   more preferably selected from the group comprising methyl, ethyl, ethyl and phenyl,
   most preferably methyl
   where the building block of the formula (I) is directly linked to an Si atom at both linkage points, and
B) crosslinked organopolysiloxanes having organopolysiloxane units bridged by a bridging siloxane of the formula (II) 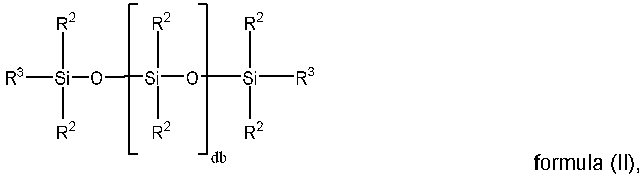 where
   R² = independently of one another, identical or different aliphatic or aromatic hydrocarbon radicals having 1 to 20 carbon atoms, preferably alkyl radicals having 1 to 14 carbon atoms, most preferably methyl,
   R³ = independently of one another, identical or different alkanediyl radical having 2 to 10, preferably 2 to 6, most preferably 2 to 3, carbon atoms,
   db = 10 to 1000, preferably 51 to 750, in particular 101 to 500,
   where the terminal carbon atoms of the bridging siloxane of the formula (II) are directly linked to an Si atom at both bridging points.

The component A) comprised in the mixture composition according to the instant invention is present in the form of a mixture with a distribution controlled essentially by laws of statistics. The values for the indices x, y and z are therefore average values. The units characterized with the indices x, y and z can be present in the compounds of the formula I in random distribution, blockwise or arranged in any other desired order.

Preferably, each linked organopolysiloxane unit in component A) has, on average, more than 1, preferably from 1 to 10, preferably from 1.1 to 5 and particularly preferably from 1.5 to 4, linkage points to a building block of the formula I.

The organopolysiloxane units in component A) comprised in the mixture composition according to the instant invention are preferably identical or different units of formula (IB)

M_{2+c+2d} Da D'_{b} T_{c} Q_{d} formula (IB),

where
- M: = (R^{1b}R^{2b}₂SiO_{1/2})
- D: = (R^{2b}₂SiO_{2/2})
- D': = (R^{2b}R^{3b}SiO_{2/2})
- T: = (R^{2b}SiO_{3/2})
- Q: = (SiO_{4/2})
- a: = 10 to 1000, preferably 40 to 500, preferably 50 to 400, particularly preferably 60 to 200,
- b: = 1 to 100, preferably 2 to 60, preferably 10 to 50,
- c: = 0 to 20, preferably 0.05 to 10, particularly preferably 0.1 to 5,
- d: = 0 to 10, preferably 0 to 5, particularly preferably 0,
- R^{1b}: = R^{2b} or R^{3b},
- R^{2b}: = independently of one another, V or H or identical or different linear or branched,
optionally aromatic hydrocarbon radicals having 1 to 32, preferably 6 to 25, carbon atoms, which optionally carry OH or ester functions, preferably C₉-, C₁₂-, C₁₆- or C₂₂-hydrocarbon radical or methyl radical or phenyl radical, in particular methyl radical,
R^{3b} = independently of one another, identical or different polyether radicals of formula (IC),

-CH₂-CH₂-(G)ₙO(EO)ₓ(PO)_{y}(XO)_{z} R^{4b} formula (IC),

where
G = divalent organic radical, preferably CH₂, C=O, CR^{5b}₂ or CHR^{5b}, preferably CH₂,
EO = (-C₂H₄O-)
PO = (-C₃H₆O-)
XO = (C₂H₃R¹O)
n = 1 to 16, preferably 1 to 9 and more preferably 1
x = 2 to 50, preferably 5 to 30, preferably from 6 to 15,
y = 0 to 50, preferably 0 or > 0 to 15, preferably 0,
z = 0 to 10, preferably 0 or > 0 to 2, preferably 0,
R¹ = independently of one another, identical or different radicals selected from the group comprising
linear or branched, saturated or unsaturated alkyl radicals having 1 to 30 carbon atoms, which are optionally interrupted by ether functions, preferably alkyl radicals having 1 to 14 carbon atoms, alkaryl radicals having 7 to 18 carbon atoms, and
aryl radicals having 6 to 16 carbon atoms,
more preferably selected from the group comprising methyl, ethyl, ethyl and phenyl,
most preferably methyl, and
R^{4b} = independently of one another, identical or different radicals selected from the group comprising H, alkyl radicals having 1 to 32 carbon atoms and carboxylate radicals, preferably comprising 1 to 22 carbon atoms, most preferably H and
V = a bond (a linkage point) to the building block of the formula (I),
where at least one V is present per organopolysiloxane unit (IB).
Preferably, radical R^{2b} is a bond V only in the units M and D. The radical R^{2b} is preferably not a hydrogen atom.

The average number of linkage points V per unit of formula (IB) is preferably more than or equal to 1, preferably 1 to 5 and more preferably 1 to 3.

It may be advantageous if, on average, at least one building block (I) is attached via an SiOC bond in the crosslinked organopolysiloxane in component A) of the instant invention per organopolysiloxane unit of the formula (IB). It may also be advantageous if, on average, at least one building block (I) is attached via an SiC bond in the crosslinked organopolysiloxane in component A) of the instant invention per organopolysiloxane unit of the formula (IB). It may be particularly advantageous if on average at least one building block (I) is attached via an SiOC bond and at least one building block (I) is attached via an SiC bond in the crosslinked organopolysiloxane in component A) of the instant invention per organopolysiloxane unit of the formula (IB), where the building block (I) is preferably not identical, i.e. a building block (I) is not bonded with an SiOC and an SiC bond at only one organopolysiloxane unit of the formula (IB).

A detailed process on how the crosslinked organopolysiloxane in component A) of the instant invention can be prepared is described in EP2301987A1.

The crosslinked organopolysiloxanes in component B) of the instant invention are preferably prepared by a method comprising the steps of
1) addition reaction of organopolysiloxanes of formula (ID) 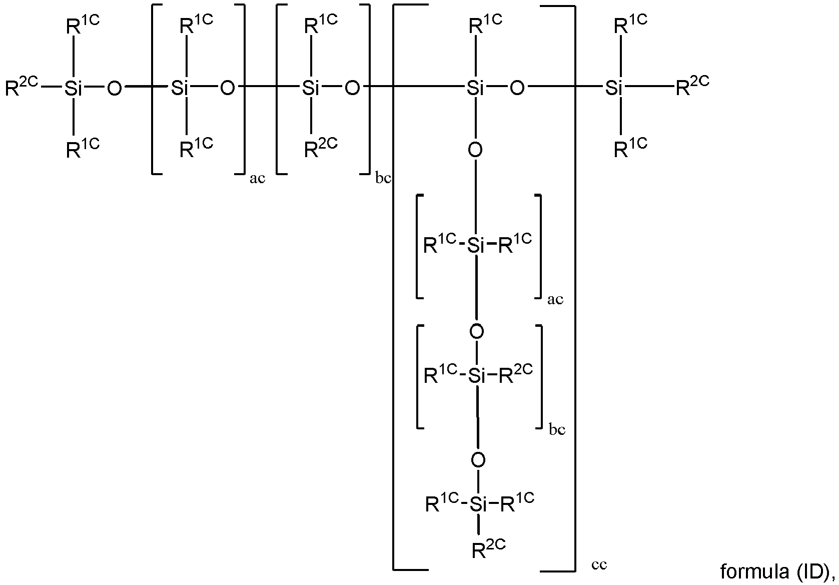 where
   R^{1c} are identical or different, branched or unbranched, aliphatic or aromatic hydrocarbon radicals having 1 to 20 carbon atoms,
   R^{2c} is R¹ or H, with the proviso that at least three radicals R^{2c} are H,
   ac is 5 to 500, preferably 10 to 250, in particular 15 to 75,
   bc is 1 to 50, preferably 1 to 20, in particular 3 to 15,
   cc is 0 to 5, preferably 0 to 1, in particular 0,
   onto siloxanes of formula (IID) containing double bonds 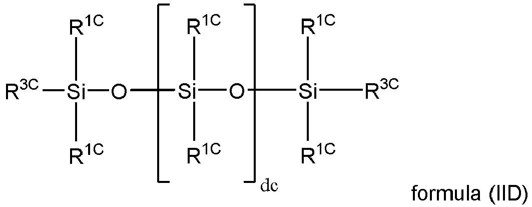 where
   dc is 10 to 1000, preferably 51 to 750, in particular 101 to 500 and
   R^{1c} as above
   R^{3c} independently of one another, are identical or different hydrocarbon radicals having 2 to 12, preferably 2 to 8, in particular 2 carbon atoms and containing at least one double bond, in the presence of platinum or rhodium catalysts, with the proviso that the organopolysiloxanes of formula (ID) are present in at least 6-fold, preferably from 6-fold to 50-fold, molar excess, based on the siloxane of formula (IID) containing double bonds, to give a reaction product having Si-H groups and with further reaction of the reaction product in at least one of the following process steps
2) transition-metal-catalysed partial or complete addition of the SiH groups onto alkenyl and/or alkynyl compounds, preferably onto double-bond-containing polyethers and α-olefins, in particular onto allyl polyethers, and/or
3) partial or complete reaction of the Si-H groups remaining after the above reaction(s) in the presence of a catalyst with at least one alcohol, from the group of linear or branched, saturated, mono- or polyunsaturated, aromatic, aliphatic-aromatic, optionally halogen-atom-containing monoalcohols, polyether monoalcohols, polyester monoalcohols, amino alcohols.

The radicals R^{1c} are preferably identical or different aliphatic or aromatic hydrocarbon radicals having 1 to 20 carbon atoms, further preferably identical or different unbranched, aliphatic or aromatic hydrocarbon radicals having 1 to 9 carbon atoms and particularly preferably methyl, ethyl or phenyl, most preferably methyl.

The proviso that the SiH-group-carrying organopolysiloxane of formula (ID) is present in at least 6-fold molar excess, based on the double bond-containing siloxane of the formula (IID), prevents the formation of a network and the formation of highly viscous products resulting. As a rule, the organosiloxanes prepared by one of the two aforementioned processes have viscosities up to 10 000 mPas. A certain fraction of the organosiloxane may be present in the product in the form of a comb-like modified siloxane.

On account of the selected reaction conditions, the double-bond-containing siloxane and the Si-functional siloxane form, in the first stage, a siloxane of the following idealized "H structure" shown in formula (III) (c = 0, R^{1C} = Me, R^{2C} = R = Me or H): 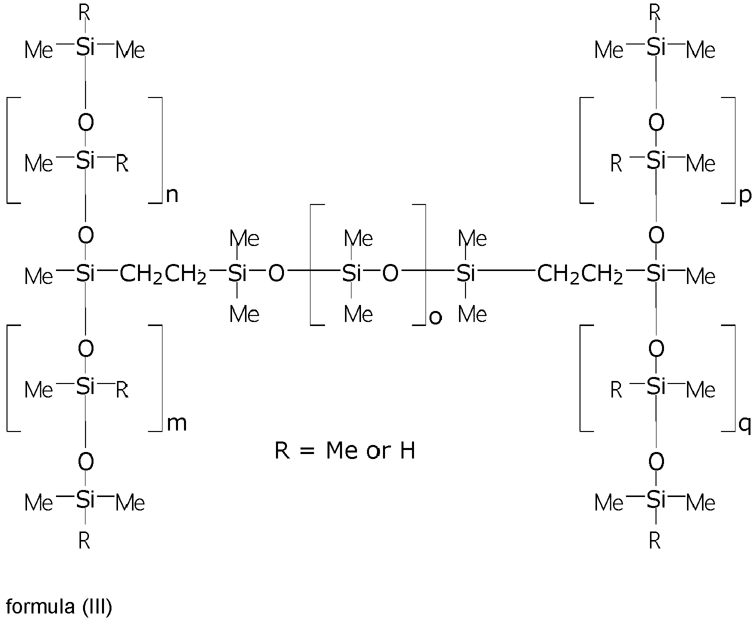 where m, n, o, p and q are positive integers.

This siloxane backbone is retained during subsequent process steps 2) and/or 3). The synthesis of the siloxane polymers can take place with or without solvents. Foaming which may possibly arise can be suppressed through the use of solvents. Suitable solvents are, for example, toluene and cyclohexane.

A detailed process on how the crosslinked organopolysiloxane in component A) of the instant invention can be prepared is described in WO2009138306.

A preferred mixture composition according to the instant invention is characterized in that the organopolysiloxane units in the crosslinked polysiloxanes in component A) are identical or different units of formula (IB)
where
- a: = 60 to 200,
- b: = 10 to 50,
- c: = 0,
- d: = 0,
- R^{1b}: = R^{2b} or R^{3b},
- R^{2b}: = independently of one another, V or H,
- R^{3b}: = independently of one another, identical or different polyether radicals of formula (IC)
where
- n: = 1,
- x: = 6 to 15,
- y: 0,
- z: = 0,
- R¹: = methyl, and
- R^{4b}: = H, and that

the crosslinked polysiloxanes in component B) are prepared by a method with organopolysiloxanes of formula (ID)
where
- R^{1c}: methyl,
- ac: is 15 to 75,
- bc: is 3 to 15,
- cc: is 0,
and with siloxanes of formula (IID)
where
- dc: 101 to 500, and
- R^{1c}: methyl
- R^{3c}: hydrocarbon radicals having 2 carbon atoms and containing at least one double bond.

It is preferred for the mixture composition according to the instant invention that component A) and B) make up at least 80 wt.-%, preferably 90 wt.-%, more preferably 95 wt.-%, of the total mixture composition.

It is preferred for the mixture composition according to the instant invention that the weight ratio of component A) to component B) in the total mixture composition is in the range from 95:5 to 5:95, preferably from 95:5 to 50:50, more preferably from 90:10 to 55:45.

It is preferred for the mixture composition according to the instant invention that component A) and component B) are crosslinked organopolysiloxanes which have been treated by hydrogenation to reduce odour often present in crosslinked organopolysiloxanes. A suited method for reducing odour by hydrogenation is disclosed, for example, in EP1970396

The invention further provides the use of a mixture composition of the instant invention as an emulsifier, more preferably as a w/o emulsifier.

The invention further provides the use of a mixture composition of the instant invention for stabilizing formulations, preferably cosmetic formulation, containing solid particles. The term "stabilizing" in this context means, that the formulations containing solid particles do not show visible phase separation after storage a 25 °C for four months.

The comprised solid particles are characterized by having a mean particle size d50 of from 0,1 to 1000 µm.

The mean particle size d50 is preferably determined by light scattering in a laser beam with a Malvern Mastersizer 2000. The determination is done using the dry measurement. Each time 20 to 40 g powder are fed using a Scirocco dry powder feeder. The particle flow is controlled operating the vibrating tray with a feed-rate of 70 %. The dispersive air pressure is adjusted to be 3 bar. Each measurement is accompanied by a measurement of the background (10 seconds / 10,000 single measurements). The measurement time of the sample is 5 seconds (5,000 single measurements). The refraction index as well as the blue light value are fixed to be 1.52. The evaluation is done using the Mie-theory.

Solid particles preferably being contained in the stabilized formulations are selected from the group comprising siloxane-particles, preferable triethoxycaprylylsilane, hydrogenated lecithin and eventually surface treated pigments, preferably selected from titanium dioxide, zink oxide, iron oxide, iron(III) oxide-hydroxide, Iron (II, III) oxide and mixtures thereof, with the cosmetic pigments Cl 77891, Cl 77947, Cl 77492, Cl 77491 and Cl 77499 being the most preferred.

The formulations in the use according to the instant invention preferably contain solid particles in an amount of from 2.0 wt.-% to 20 wt.-%, preferably from 7.0 wt.-% to 14 wt.-%, wherein the weight percentages refer to the total formulation.

The mixture composition is preferably used according to the instant invention in an amount of from 1.0 wt.-% to 5.0 wt.-%, preferably from 2.0 wt.-% to 3.0 wt.-%, wherein the weight percentages refer to the total formulation.

Furthermore, the formulations in the use according to the instant invention can comprise at least one additional component selected from the group of
emollients,
emulsifiers,
thickeners/viscosity regulators/stabilizers,
UV photoprotective filters,
antioxidants,
hydrotropes (or polyols),
film formers,
pearlescent additives,
deodorant and antiperspirant active ingredients,
insect repellents,
self-tanning agents,
preservatives,
conditioners,
perfumes,
dyes,
odour absorbers,
cosmetic active ingredients,
care additives,
superfatting agents,
solid particles,
solvents,
wherein perfums, and/or UV photoprotective filters, especially organic filters, are preferably comprised.

Substances which can be used as exemplary representatives of the individual groups to be comprised in the formulation according to the invention are known to the person skilled in the art and can be found for example in the German application DE 102008001788.4. This patent application is hereby incorporated by reference and thus forms part of the disclosure.

As regards further optional components and the amounts of these components used, reference is made expressly to the relevant handbooks known to the person skilled in the art, for example K. Schrader, "Grundlagen und Rezepturen der Kosmetika [Fundamentals and Formulations of Cosmetics]", 2nd edition, page 329 to 341, Hüthig Buch Verlag Heidelberg.

The amounts of the respective additives are dependent on the intended use.

Typical guide formulations for the respective applications are known prior art and are contained for example in the brochures of the manufacturers of the respective base materials and active ingredients. These existing formulations can generally be adopted unchanged. If required, however, the desired modifications can be undertaken without complication by means of simple experiments for the purposes of adaptation and optimization.

Instantly claimed is also the use of a mixture composition of the instant invention for the dispersion of solid pigments. The solid pigments are those solid particles comprised in the formulation in the use described above of the present invention.

The present invention is described by way of example in the examples listed below, without any intention to limit the invention, the scope of application of which arises from the entire description and the claims, to the embodiments specified in the examples.

### Examples:

### Example 1: Emulsifier 1

### Component A):

In a multineck flask rendered inert with argon and equipped with precision-ground glass stirrer, dropping funnel and reflux condenser, 1675 g of a siloxane carrying pendant SiH groups and of the average composition MD₇₅(DH)₂₅M (SiH content: 3.6 mol/kg) were admixed at 21 °C with 5.025 ml of the catalyst solution described in EP-B-1 520 870 (12 wppm Pt).

Over the course of 40 minutes, 586.7 g of 1-hexadecene were added dropwise such that the heat of reaction increased the starting temperature to 66°C. Over the course of 30 minutes, 993.6 g of a polyether of average composition CH₂=CH-CH₂O-(C₂H₄O)₈-H were then quickly added dropwise, during which the reaction temperature was kept at a maximum of 56°C. When the addition was complete, a further 304.8 g of 1-hexadecene were added over the course of 20 minutes. The mixture was immediately cooled to 20°C after the metered addition. The conversion of the SiH-groups was 92.7%. The viscosity was 1165 mPas.

The product from the above reaction (1.8 kg) was heated to 110°C, admixed with 125 ppm of trispentafluorophenylborane (dissolved in allyl polyether solution) and stirred for 1 h. The resulting reaction product was then diluted (1:1) with the starting material (product from the above reaction) and cooled. Following the addition of 50 ppm of triisopropanolamine, the mixture was stirred for a further 30 min and then the high-viscosity product was drawn off. The viscosity was 3636 mPas.

### Component B):

Firstly, 183 g of an SiH-functional siloxane of the general formula
Me₃SiO(SiMe₂O)₂₈(SiMeHO)₁₀SiMe₃ were mixed with 143 g of a vinylsiloxane of the general formula CH₂=CH-SiMe₂O-(SiMe₂O)₃₄₈-SiMe₂-CH=CH₂, heated to 90°C and treated with 5 ppm of an Pt catalyst. After 30 min, 1322 g of an allyl polyether of the general formula CH₂=CH-CH₂-O-(CH₂CH₂O)₂₅(CH₂CH(CH₃)O)₄Me were then added, the mixture was heated to 100 °C. The mixture was then further stirred for 3 h at 100°C until a complete reaction of all SiH groups was reached.

### Emulsifier 1:

Component A) and B) from above were mixed in a weight ratio of 60:40, respectively, by stirring for 30 minutes at 22 °C.

### Example 2: Emulsifier 2

### Component A):

See example 1.

### Component B):

Firstly, 224 g of an SiH-functional siloxane of the general formula
Me₃SiO(SiMe₂O)₄₃(SiMeHO)₅SiMe₃ were mixed with 143 g of a vinylsiloxane of the general formula CH₂=CH-SiMe₂O-(SiMe₂O)₁₄₈-SiMe₂-CH=CH₂, heated to 90°C and treated with 5 ppm of an Pt catalyst. After 30 min, 609 g of an allyl polyether of the general formula CH₂=CH-CH₂-O-(CH₂CH₂O)₁₃(CH₂CH(CH₃)O)₁₄Me were then added, the mixture was heated to 100 °C. The mixture was then further stirred for 3 h at 100°C until a complete reaction of all SiH groups was reached.

### Emulsifier 2:

Component A) and B) from above were mixed in a weight ratio of 80:20, respectively, by stirring for 30 minutes at 22 °C.

### Example 3: Emulsion and pigment stability

The emulsifier blend described in this invention allows the stabilisation of versatile pigments in cosmetic dispersion even at a high solid content. This is shown in the formulations listed in the table below.

The stability was measured by centrifugation of 16 g foundation for 90 min at 1.7 rcf and additional 30 min at 3.0 rcf.

For evaluation, oil and pigment separation were quantified. The following terminology was used to describe the stability:

| | **Emulsion stability** | **Pigment stability** |
|---|---|---|
| **Very good (++)** | No or minimal oil separation | Colour is homogenous |
| **Good (+)** | Very weak oil separation | Colour is slightly inhomogeneous |
| **Medium (0)** | Weak oil separation | Pigment phase weakly visible; rest homogenous |
| **Weak (-)** | Strong oil separation | Distinct pigment phase visible; rest homogenous |
| **Very weak (--)** | Very strong oil separation | Strong pigment sedimentation |

The preparation of each formulation was performed by addition of the water phase (A) into the oil phase (B) and subsequent homogenization using common methods.

### 1. W/O Foundation with high pigment-load (make-up)

| | **Nomenclature according to INCI** | **1** | **2** | **3** | **4** | **5** | **6** |
|---|---|---|---|---|---|---|---|
| A | Example 2, according to invention | 3.00 | | | | | |
| | Component A of example 2 | | 3.00 | | | | |
| | Component B of example 2 | | | 3.00 | | | |
| | Polysiloxane No.3 ¹⁾ | | | | 3.00 | | |
| | Polysiloxane No.4 ²⁾ | | | | | 3.00 | |
| | Polysiloxane blend No.5 ³⁾ | | | | | | 5.50 |
| | Polyglyceryl-4 Diisostearate/ Polyhydroxystearate/ Sebacate ⁴⁾ | 1.00 | 1.00 | 1.00 | 1.00 | 1.00 | 1.00 |
| | Dimethicone ⁵⁾ | 13.6 | 13.6 | 13.6 | 13.6 | 13.6 | 12.1 |
| | Butylene Glycol Dicaprylate/Dicaprat ⁶⁾ | 6.00 | 6.00 | 6.00 | 6.00 | 6.00 | 6.00 |
| | Decyl Cocoate ⁷⁾ | 4.00 | 4.00 | 4.00 | 4.00 | 4.00 | 4.00 |
| | Isoamyl Laurate ⁸⁾ | 5.00 | 5.00 | 5.00 | 5.00 | 5.00 | 5.00 |
| | Cl 77891 (and) Triethoxycaprylylsilane ⁹⁾ | 12.0 | 12.0 | 12.0 | 12.0 | 12.0 | 12.0 |
| | Cl 77492 (and) Triethoxycaprylylsilane ¹⁰⁾ | 1.20 | 1.20 | 1.20 | 1.20 | 1.20 | 1.20 |
| | Cl 77491 (and) Triethoxycarpylylsilane ¹¹⁾ | 0.50 | 0.50 | 0.50 | 0.50 | 0.50 | 0.50 |
| | Cl 77499 (and) Triethoxycaprylylsilane ¹²⁾ | 0.30 | 0.30 | 0.30 | 0.30 | 0.30 | 0.30 |
| | Disteardimonium Hectorite dimethicone, propylencarbonat ¹³⁾ | 2.00 | 2.00 | 2.00 | 2.00 | 2.00 | 2.00 |
| B | Water | ad 100 | ad 100 | ad 100 | ad 100 | ad 100 | ad 100 |
| | Sodium Chloride | 1.00 | 1.00 | 1.00 | 1.00 | 1.00 | 1.00 |
| | Glycerin | 3.00 | 3.00 | 3.00 | 3.00 | 3.00 | 3.00 |
| C | Phenoxyethanol, Caprylyl Glycol ¹⁴⁾ | 1.00 | 1.00 | 1.00 | 1.00 | 1.00 | 1.00 |
| | Emulsion stability: | ++ | + | - | 0 | + | 0 |
| | Pigment stability: | ++ | ++ | - | - | ++ | 0 |

| | | | | | | | |
|---|---|---|---|---|---|---|---|
| ¹⁾ ABIL^{®} EM 90 (Evonik Operations GmbH) ²⁾ KF-6038 (Shin-Etsu Silicones of America) ³⁾ ABIL^{®} WE 09 (Evonik Operations GmbH) ⁴⁾ Isolan^{®} GPS (Evonik Operations GmbH) ⁵⁾ Belsil^{®} DM 5 (Wacker Chemie AG) ⁶⁾ Dermofeel^{®} BGC (Evonik Operations GmbH) ⁷⁾ Tegosoft^{®} DC (Evonik Operations GmbH) ⁸⁾ Dermofeel^{®} Sensolv (Evonik Operations GmbH) ⁹⁾ Unipure^{®} White LC 987 AS-EM (Sensient Cosmetic Technologies) ¹⁰⁾ Unipure^{®} Yellow LC 182 AS-EM (Sensient Cosmetic Technologies) ¹¹⁾ Unipure^{®} Red LC 381 AS-EM (Sensient Cosmetic Technologies) ¹²⁾ Unipure^{®} Black LC 989 AS-EM (Sensient Cosmetic Technologies) ¹³⁾ Bentone^{®} 38V CG (Elementis) ¹⁴⁾ Verstatil^{®} PC (Evonik Dr. Straetmans GmbH) | | | | | | | |

### Further example formulations

The following examples demonstrate the versatile applicability of the polysiloxane blend in different cosmetic formulations and their compatibility with versatile oils as well as with active compounds like UV-filters or antimicrobial drugs which are usually challenging to formulate. The application of the invention is not limited to the formulations shown.

### W/O Foundation with low pigment-load (make-up)

| | **Nomenclature according to INCI** | **6** | **7** | **8** | **9** | **10** |
|---|---|---|---|---|---|---|
| **A** | Example 2, according to invention | 3.00 | 3.00 | 2.00 | 3.00 | 2.50 |
| | Polyglyceryl-4 Diisostearate/ Polyhydroxystearate/ Sebacate ¹⁾ | 1.00 | 1.00 | 3.00 | | |
| | Polyglyceryl-2 Dipolyhydroxystearate ²⁾ | | | | 1.50 | |
| | Polyglyceryl-3 Polyricinoleate ³⁾ | | | | | 2.00 |
| | Isoamyl Laurate ⁴⁾ | 7.00 | 6.00 | 5.00 | 7.00 | 2.50 |
| | Decyl Cocoate ⁵⁾ | 9.00 | 8.00 | | 9.00 | |
| | Isopropyl Myristate ⁶⁾ | 6.00 | | | 6.00 | 4.50 |
| | Caprylic/Capric Triglyceride ⁷⁾ | | 4.00 | | | 3.00 |
| | Diethylhexyl Carbonate ⁸⁾ | | | 4.00 | | 5.00 |
| | Dimethicone ⁹⁾ | | | 15.00 | | |
| | Cl 77891 (and) Hydrogenated Lecithin ¹⁰⁾ | 5.00 | 5.00 | 5.00 | 5.00 | 5.00 |
| | Cl 77492 (and) Hydrogenated Lecithin ¹¹⁾ | 0.60 | 0.60 | 0.60 | 0.60 | 0.60 |
| | Cl 77491 (and) Hydrogenated Lecithin ¹²⁾ | 0.35 | 0.35 | 0.35 | 0.35 | 0.35 |
| | Cl 77499 (and) Hydrogenated Lecithin ¹³⁾ | 0.10 | 0.10 | 0.10 | 0.10 | 0.10 |
| | Disteardimonium Hectorite dimethicone, propylencarbonat ¹⁴⁾ | 2.00 | 2.00 | 2.00 | 2.00 | 2.00 |
| **B** | Water | ad 100 | ad 100 | ad 100 | ad 100 | ad 100 |
| | Sodium chloride | 1.00 | 1.00 | 1.50 | | |
| | Magnesium Sulfate Heptahydrate | | | | 1.50 | 2.00 |
| | Glycerin | 3.00 | 3.00 | 3.00 | 5.00 | 5.00 |
| **C** | Phenoxyethanol, Caprylyl Glycol ¹⁵⁾ | 1.00 | 1.00 | 1.00 | 1.00 | 1.00 |

| | | | | | | |
|---|---|---|---|---|---|---|
| ¹⁾ Isolan^{®} GPS (Evonik Operations GmbH) ²⁾ Dehymuls^{®} PGPH (BASF SE) ³⁾ Cithrol PG3PR (Croda International plc.) ⁴⁾ Dermofeel^{®} Sensolv (Evonik Operations GmbH) ⁵⁾ Tegosoft^{®} DC (Evonik Operations GmbH) ⁶⁾ Tegosoft^{®} M (Evonik Operations GmbH) ⁷⁾ Tegosoft^{®} CT (Evonik Operations GmbH) ⁸⁾ Tegosoft^{®} DEC (Evonik Operations GmbH) ⁹⁾ Belsil^{®} DM 5 (Wacker Chemie AG) ¹⁰⁾ Unipure White LC 981 HLC (Sensient Cosmetic Technologies) ¹¹⁾ Unipure Yellow LC 182 HLC (Sensient Cosmetic Technologies) ¹²⁾ Unipure Red LC 381 HLC (Sensient Cosmetic Technologies) ¹³⁾ Unipure Black LC 989 HLC (Sensient Cosmetic Technologies) ¹⁴⁾ Bentone^{®} Bentonepaste II 15) Verstatil^{®} PC (Evonik Dr. Straetmans GmbH) | | | | | | |

### Sunscreen with inorganic UV-filters:

| | **Nomenclature according to INCI** | **11** | **12** | **13** | **14** | **15** |
|---|---|---|---|---|---|---|
| **A** | Example 2, according to invention | 3.00 | 3.00 | 3.00 | 2.00 | 1.50 |
| | Polyglyceryl-4 Diisostearate/ Polyhydroxystearate/ Sebacate ¹⁾ | 1.00 | 1.00 | 1.00 | | |
| | PEG-30 Dipolyhydroxystearate ²⁾ | | | | 2.00 | |
| | Lauryl PEG-10 Tris(Trimethylsiloxy)silylethyl Dimethicone ³⁾ | | | | | 2.00 |
| | Isoamyl Laurate ⁴⁾ | 6.00 | 6.00 | 8.00 | | 3.00 |
| | Decyl Cocoate ⁵⁾ | 9.00 | | 8.00 | 2.50 | |
| | Diethylhexyl Carbonate ⁶⁾ | | 8.00 | | 5.00 | 6.50 |
| | Phenoxyethyl Caprylate ⁷⁾ | 8.00 | 8.00 | 8.00 | | |
| | C12-15 Alkyl Benzoate ⁸⁾ | | | | 10.00 | 8.00 |
| | Zink oxide | 13.0 | 13.00 | 13.00 | 8.00 | 11.00 |
| | Titanium Dioxide (and) Silica (and) Dimethicone ⁹⁾ | 6.00 | 6.00 | 6.00 | 10.00 | 7.00 |
| | Triethyl Citrate; Caprylyl Glycol; Benzoic Acid ¹⁰⁾ | 1.00 | 1.00 | 1.00 | 1.00 | 1.00 |
| **B** | Glycerin | 5.00 | 5.00 | 5.00 | 3.00 | 2.00 |
| | Zinc Sulfate Heptahydrate | 1.50 | 1.50 | 1.50 | 2.00 | 2.00 |
| | Water | ad 100 | ad 100 | ad 100 | ad 100 | ad 100 |

| | | | | | | |
|---|---|---|---|---|---|---|
| ¹⁾ Isolan^{®} GPS (Evonik Operations GmbH) ²⁾ Cithrol DPHS (Croda International pic.) ³⁾ Dowsil ES-5300 Formulation Aid (Dow) ⁴⁾ Dermofeel^{®} Sensolv (Evonik Operations GmbH) ⁵⁾ Tegosoft^{®} DC (Evonik Operations GmbH) ⁶⁾ Tegosoft^{®} DEC (Evonik Operations GmbH) ⁷⁾ Tegosoft^{®} XC (Evonik Operations GmbH) ⁸⁾ Tegosoft^{®} TN (Evonik Operations GmbH) ⁹⁾ Parsol^{®} TX (DSM Nutritional Products Europe Ltd) ¹⁰⁾ Versatil^{®} TBO (Evonik Dr. Straetmans GmbH) | | | | | | |

### Sunscreen with organic UV-filters:

| | **Nomenclature according to INCI** | **16** | **17** | **18** | **19** | **20** |
|---|---|---|---|---|---|---|
| **A** | Example 2, according to invention | 3.00 | 3.00 | 3.00 | 2.00 | 2.50 |
| | Polyglyceryl-4 Diisostearate/ Polyhydroxystearate/ Sebacate ¹⁾ | 1.00 | 1.00 | 1.00 | 0.50 | |
| | Cetyl Diglyceryl Tris(Trimethylsiloxy)silylethyl Dimethicone ²⁾ | | | | 1.50 | |
| | Lauryl PEG-9 Polydimethylsiloxyethyl Dimethicone ³⁾ | | | | | 2.00 |
| | Diethylhexyl Carbonate ⁴⁾ | 5.00 | | 5.00 | 5.00 | 5.00 |
| | Isoamyl Laurate ⁵⁾ | | 5.00 | | | |
| | Phenoxyethyl Caprylate ⁶⁾ | 5.00 | 5.00 | | | 4.50 |
| | C12-15 Alkyl Benzoate ⁷⁾ | | | 5.00 | 5.00 | |
| | Ethylhexyl Triazone ⁸⁾ | 2.00 | 2.00 | 2.00 | 2.00 | 2.00 |
| | Bis-Ethylhexyloxyphenol Methoxyphenyl triazine ⁹⁾ | 6.00 | 6.00 | 6.00 | 6.00 | 6.00 |
| | Diethylamino Hydroxybenzoyl Hexyl Benzoate (DHHB) ¹⁰⁾ | 4.00 | 4.00 | 4.00 | 4.00 | 4.00 |
| | Ethylhexyl Salicylate ¹¹⁾ | 4.00 | 4.00 | 4.00 | 4.00 | 4.00 |
| | Homomenthyl Salicylate ¹²⁾ | 4.00 | 4.00 | 4.00 | 4.00 | 4.00 |
| | Cera Alba (Bee wax) | 0.50 | | | | |
| | Cera Microcristallina | | 0.60 | 0.60 | 0.60 | 0.60 |
| | Hydrogenated Castor Oil (Castor wax) | 0.50 | 0.40 | 0.40 | 0.40 | 0.40 |
| **B** | Glycerin | 4.00 | 5.00 | 5.00 | 3.00 | 2.00 |
| | Sodium Chloride | 1.50 | 1.50 | 1.50 | 1.50 | 1.50 |
| | Water | ad 100 | ad 100 | ad 100 | ad 100 | ad 100 |
| | Disodium Ethylenediaminetetraacetic Acid (EDTA) | 0.05 | 0.05 | 0.05 | 0.05 | 0.05 |
| | Methylpropandiol, Caprylyl Glycol, Phenylpropanol ¹³) | 3.00 | 3.00 | 3.00 | 3.00 | 3.00 |

| | | | | | | |
|---|---|---|---|---|---|---|
| ¹⁾ Isolan^{®} GPS (Evonik Operations GmbH) ²⁾ Dowsil ES-5600 Silicone Glycerol Emulsifier (Dow) ³⁾ KF-6038 (Shin-Etsu Silicones of America) ⁴⁾ Tegosoft^{®} DEC (Evonik Operations GmbH) ⁵⁾ Dermofeel^{®} Sensolv (Evonik Operations GmbH) ⁶⁾ Tegosoft^{®} XC (Evonik Operations GmbH) ⁷⁾ Tegosoft^{®} TN Evonik Operations GmbH) ⁸⁾ Uvinul^{®} T 150 (BASF SE) ⁹⁾ Tinosorb^{®} S (BASF SE) ¹⁰⁾ Uvinul^{®} A plus Granular (BASF SE) ¹¹⁾ Neo Helipan^{®} OS (Symrise) ¹²⁾ Neo Helipan^{®} HMS (Symrise) ¹³⁾ Dermosoft OMP (Evonik Operations GmbH) | | | | | | |

### Rich O/W soft creme:

| | **Nomenclature according to INCI** | **21** | **22** | **23** | **24** | **25** | |
|---|---|---|---|---|---|---|---|
| **A** | Example 2, according to invention | 2.00 | 2.00 | 2.00 | 1.50 | 1.00 | 1.50 |
| | Polyglyceryl-3 Polydimethylsiloxyethyl Dimethicone ¹⁾ | | | | 1.50 | | |
| | Diisostearoyl Polyglyceryl-3 Dimer Dilinoleate ²⁾ | | | | | 1.50 | |
| | Polyglyceryl-2 Sesquiisostearate ³⁾ | | | | | | 1.00 |
| | Isoamyl Laurate ⁴⁾ | 5.40 | 5.40 | 5.40 | 3.00 | | 5.00 |
| | Dimethicone 1 ⁵⁾ | 1.00 | 1.00 | 1.00 | 2.00 | 3.00 | |
| | Cetyl Dimethicone ⁶⁾ | 1.60 | 1.60 | 1.60 | | 1.50 | 2.00 |
| | Dimethicone 2 ⁷⁾ | 11.00 | 9.60 | 9.60 | 6.00 | 8.00 | 3.00 |
| | Mineral Oil | | | | 5.00 | 2.00 | 5.00 |
| | Zinc Stearate | 0.60 | | | | | |
| | Hydrogenated Castor Oil | | 0.80 | 0,80 | 1.00 | | 0.50 |
| | Cera Microcristallina | | 1.20 | | 1.00 | 2.00 | |
| | Cera Alba | | | 1.20 | | | 1.50 |
| | Cetyl Ricenoleate | 1.00 | 1.00 | 1.00 | | | 1.00 |
| **B** | Glycerin | 3.00 | 3.00 | 3.00 | 3.00 | 3.00 | 4.00 |
| | Sodium Chloride | 1.00 | 1.00 | 1.00 | 1.00 | 1.00 | |
| | Water | ad 100 | ad 100 | ad 100 | ad 100 | ad 100 | |
| | 1,2-Hexanediol ⁸⁾ | 2.00 | 2.00 | 2.00 | 2.00 | 2.00 | 2.00 |

| | | | | | | | |
|---|---|---|---|---|---|---|---|
| ¹⁾ KF-6104 (Shin-Etsu Silicones of America) ²⁾ Isolan^{®} PDI (Evonik Operations GmbH) ³⁾ Plantasens Emulsifier DGI (Clariant) ⁴⁾ Dermofeel^{®} Sensolv (Evonik Operations GmbH) ⁵⁾ ABIL^{®}350 (Evonik Operations GmbH) ⁶⁾ ABIL^{®}WAX 9801 (Evonik Operations GmbH) ⁷⁾ Belsil^{®} DM 5 (Wacker Chemie AG) ⁸⁾ Dermosoft Hexiol (Evonik Operations GmbH) | | | | | | | |

## Claims

1. Mixture composition comprising
A) crosslinked organopolysiloxanes having organopolysiloxane units linked by a building block of the formula (I)
-CH₂-CH₂-(G)ₙO(EO)ₓ(PO)_{y}(XO)_{z}- formula (I),
where
G = divalent organic radical, preferably CH₂, C=O, CR¹₂ or CHR¹, preferably CH₂,
EO = C₂H₄O
PO = C₃H₆O
XO = C₂H₃R¹O
n = 1 to 16, preferably 1 to 9 and more preferably 1
x = 2 to 50, preferably 5 to 30, preferably from 6 to 15,
y = 0 to 50, preferably 0 or > 0 to 15, preferably 0,
z = 0 to 10, preferably 0 or > 0 to 2, preferably 0,
R¹ = independently of one another, identical or different radicals selected from the group comprising linear or branched, saturated or unsaturated alkyl radicals having 1 to 30 carbon atoms, which are optionally interrupted by ether functions, preferably alkyl radicals having 1 to 14 carbon atoms, alkaryl radicals having 7 to 18 carbon atoms, and aryl radicals having 6 to 16 carbon atoms, more preferably selected from the group comprising methyl, ethyl, ethyl and phenyl, most preferably methyl,
where the building block of the formula (I) is directly linked to an Si atom at both linkage points, and
B) crosslinked organopolysiloxanes having organopolysiloxane units bridged by a bridging siloxane of the formula (II) 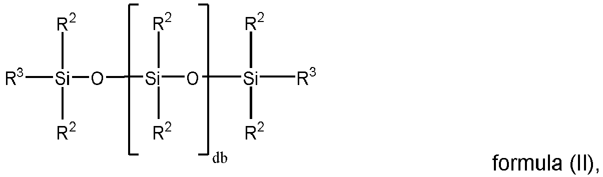 where
R² = independently of one another, identical or different aliphatic or aromatic hydrocarbon radicals having 1 to 20 carbon atoms, preferably alkyl radicals having 1 to 14 carbon atoms, most preferably methyl,
R³ = independently of one another, identical or different alkanediyl radical having 2 to 10, preferably 2 to 6, most preferably 2 to 3, carbon atoms,
db = 10 to 1000, preferably 51 to 750, in particular 101 to 500,
where the terminal carbon atoms of the bridging siloxane of the formula (II) are directly linked to an Si atom at both bridging points.

2. Mixture composition according to claim 1 **characterized in that** the organopolysiloxane units in the crosslinked polysiloxanes in component A) are identical or different units of formula (IB)
M_{2+c+2d} Dₐ D'_{b} T_{c} Q_{d} formula (IB),
where
M = (R^{1b}R^{2b}₂SiO_{1/2})
D = (R^{2b}₂SiO_{2/2})
D' = (R^{2b}R^{3b}SiO_{2/2})
T = (R^{2b} Si O_{3/2})
Q = (Si O_{4/2})
a = 10 to 1000, preferably 40 to 500, preferably 50 to 400, particularly preferably 60 to 200,
b = 1 to 100, preferably 2 to 60, preferably 10 to 50,
c = 0 to 20, preferably 0.05 to 10, particularly preferably 0.1 to 5,
d = 0 to 10, preferably 0 to 5, particularly preferably 0,
R^{1b} = R^{2b} or R^{3b},
R^{2b} = independently of one another, V or H or identical or different linear or branched, optionally aromatic hydrocarbon radicals having 1 to 32, preferably 6 to 25, carbon atoms, which optionally carry OH or ester functions, preferably C₉-, C12-, C₁₆- or C22-hydrocarbon radical or methyl radical or phenyl radical, in particular methyl radical,
R^{3b} = independently of one another, identical or different polyether radicals of formula (IC)
-CH₂-CH₂-(G)ₙO(EO)ₓ(PO)_{y}(XO)_{z} R^{4b} formula (IC),
where
G = divalent organic radical, preferably CH₂, C=O, CR^{5b}₂ or CHR^{5b}, preferably CH₂,
EO = (-C₂H₄O-)
PO = (-C₃H₆O-)
XO = (C₂H₃R¹O)
n = 1 to 16, preferably 1 to 9 and more preferably 1
x = 2 to 50, preferably 5 to 30, preferably from 6 to 15,
y = 0 to 50, preferably 0 or > 0 to 15, preferably 0,
z = 0 to 10, preferably 0 or > 0 to 2, preferably 0,
R¹ = independently of one another, identical or different radicals selected from the group comprising
linear or branched, saturated or unsaturated alkyl radicals having 1 to 30 carbon atoms, which are optionally interrupted by ether functions, preferably alkyl radicals having 1 to 14 carbon atoms, alkaryl radicals having 7 to 18 carbon atoms, and
aryl radicals having 6 to 16 carbon atoms,
more preferably selected from the group comprising methyl, ethyl, ethyl and phenyl,
most preferably methyl, and
R^{4b} = independently of one another, identical or different radicals selected from the group comprising H, alkyl radicals having 1 to 32 carbon atoms and carboxylate radicals, preferably comprising 1 to 22 carbon atoms, most preferably H, and
V = a bond (a linkage point) to the building block of the formula (I),
where at least one V is present per organopolysiloxane unit (IB).

3. Mixture composition according to claim 1 or 2 **characterized in that** the crosslinked polysiloxanes in component A) bear an average number of linkage points V per unit of formula (IB) of more than or equal to 1, preferably of 1 to 5 and more preferably of 1 to 3.

4. Mixture composition according to at least one of the preceding claims **characterized in that** the crosslinked polysiloxanes in component B) are prepared by a method comprising the steps of
1) addition reaction of organopolysiloxanes of formula (ID) 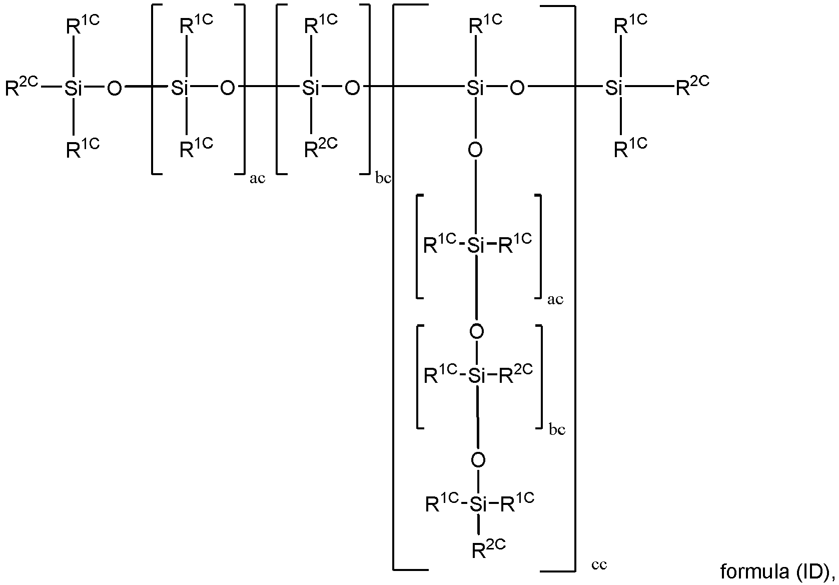 where
R^{1c} are identical or different, branched or unbranched, aliphatic or aromatic hydrocarbon radicals having 1 to 20 carbon atoms,
R^{2c} is R¹ or H, with the proviso that at least three radicals R^{2c} are H,
ac is 5 to 500, preferably 10 to 250, in particular 15 to 75,
bc is 1 to 50, preferably 1 to 20, in particular 3 to 15,
cc is 0 to 5, preferably 0 to 1, in particular 0,
onto siloxanes of formula (IID) containing double bonds 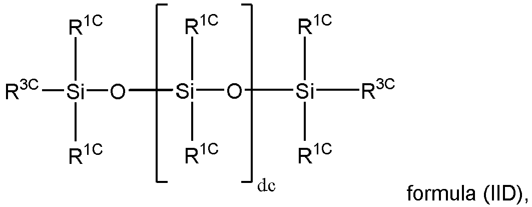 where
dc is 10 to 1000, preferably 51 to 750, in particular 101 to 500 and
R^{1c} as above
R^{3c} independently of one another, are identical or different hydrocarbon radicals having 2 to 12, preferably 2 to 8, in particular 2 carbon atoms and containing at least one double bond,
in the presence of platinum or rhodium catalysts, with the proviso that the organopolysiloxanes of formula (ID) are present in at least 6-fold molar excess, based on the siloxane of formula (IID) containing double bonds, to give a reaction product having Si-H groups and with further reaction of the reaction product in at least one of the following process steps,
2) transition-metal-catalysed partial or complete addition of the SiH groups onto alkenyl and/or alkynyl compounds, preferably onto double-bond-containing polyethers and α olefins, in particular onto allyl polyethers, and/or
3) partial or complete reaction of the Si-H groups remaining after the above reaction(s) in the presence of a catalyst with at least one alcohol, from the group of linear or branched, saturated, mono- or polyunsaturated, aromatic, aliphatic-aromatic, optionally halogen-atom-containing monoalcohols, polyether monoalcohols, polyester monoalcohols, amino alcohols.

5. Mixture composition according to claim 4 **characterized in that** the crosslinked polysiloxane in component B) the radicals R^{1c} are identical or different unbranched, aliphatic or aromatic hydrocarbon radicals having 1 to 9 carbon atoms and preferably methyl, ethyl or phenyl, most preferably methyl.

6. Mixture composition according to claim 4 or 5 **characterized in that**
the organopolysiloxane units in the crosslinked polysiloxanes in component A) are identical
or different units of formula (IB)
where
a = 60 to 200,
b = 10 to 50,
c = 0,
d = 00,
R^{1b} = R^{2b} or R^{3b},
R^{2b} = independently of one another, V or H,
R^{3b} = independently of one another, identical or different polyether radicals of formula (IC)
where
n = 1,
x = 6 to 15,
y 0,
z = 0,
R¹ = methyl, and
R^{4b} = H, and that
the crosslinked polysiloxanes in component B) are prepared by a method with
organopolysiloxanes of formula (ID)
where
R^{1c} methyl,
ac is 15 to 75,
bc is 3 to 15,
cc is 0,
and with siloxanes of formula (IID)
where
dc 101 to 500, and
R^{1c} methyl
R^{3c} hydrocarbon radicals having 2 carbon atoms and containing at least one double bond.

7. Mixture composition according to at least one of the preceding claims **characterized in that** component A) and B) make up at least 80 wt.-%, preferably 90 wt.-%, more preferably 95 wt.-%, of the total mixture composition.

8. Mixture composition according to at least one of the preceding claims **characterized in that** the weight ratio of component A) to component B) in the total mixture composition is in the range from 95:5 to 5:95, preferably from 95:5 to 50:50, more preferably from 90:10 to 55:45.

9. Use of a mixture composition according to at least one of the preceding claims as an emulsifier, more preferably as emulsifier, more preferably as a w/o emulsifier.

10. Use of a mixture composition according to at least one of the claims 1 to 8 for stabilizing formulations, preferably cosmetic formulation, containing solid particles.

11. Use according to claim 10 **characterized in that** the formulations contains solid particles in an amount of from 2.0 wt.-% to 20 wt.-%, preferably from 7.0 wt.-% to 14 wt.-%, wherein the weight percentages refer to the total formulation

12. Use according to claim 10 or 11 **characterized in that** mixture composition is used in an in an amount of from 1.0 wt.-% to 5.0 wt.-%, preferably from 2.0 wt.-% to 3.0 wt.-%, wherein the weight percentages refer to the total formulation.

13. Use of a mixture composition according to at least one of the claims 1 to 8 for the dispersion of solid pigments.
